# EUROPEAN PATENT APPLICATION

(11) **EP 4 574 845 A1**
(43) Date of publication of application: **25.06.2025**
(21) Application number: 23854226.0
(22) Date of filing: 31.07.2023
(51) Int. Cl.: C07K 19/00, C12N 15/62, C12N 15/63, A61K 38/20, A61P 35/00, A61P 37/00

(54) **IL-15 MUTANT-FC/IL-15R-ALPHA SUBUNIT-FC HETERODIMER AND USE THEREOF**

(30) Priority: 15.08.2022 CN 202210976058
(71) Applicant: Yifan Pharmaceutical (Shanghai) Co., Ltd., Shanghai 201615 (CN)
(72) Inventor: ZHOU, Haiping, Beijing 101318 (CN); HONG, Haiyan, Beijing 101318 (CN); ZHANG, Xueliang, Beijing 101318 (CN); LI, Chongyang, Beijing 101318 (CN); TAN, Xiaolu, Beijing 101318 (CN)
(74) Representative: ABG Intellectual Property Law, S.L.
(86) International application number: PCT/CN2023/110205
(87) International publication number: WO 2024/037322

(57) **Abstract**

The invention discloses an IL-15 mutant-Fc/IL-15Rα subunit-Fc heterodimer and use thereof. The heterodimer protein comprises protein a and protein b, wherein the protein a comprises an IL-15 mutant and a first Fc mutant; the protein b comprises a sushi domain of an IL-15Rα subunit and a fragment of the other part of the IL-15Rα subunit and a second Fc mutant; the first Fc mutant and the second Fc mutant are selected from a Knob modified Fc or a Hole modified Fc, and the modification types of the two Fc mutants are different. It showed in experiments that the heterodimer protein provided by the invention could effectively stimulate the proliferation of NK cells and T cells, and in vivo experiments showed that the heterodimer protein HL-015-9 was superior to ALT-803 on the inhibition of growth of tumors at each equivalent injected dose, and could be used for preparing medicaments or formulations for treating tumors and/or viral infections, among other diseases.

## Description

### Technical Field

The invention is in the technical field of medical formulations, and particularly relates to an IL-15 mutant-Fc/IL-15Rα subunit-Fc heterodimer and use thereof.

### Background Art

Human interleukin-15 (IL-15) is a pleiotropic cytokine that functions to activate T cells, B cells and NK cells and to mediate proliferation and survival of these cells. In addition, IL-15 is capable of activating, maintaining, and expanding CD8+ memory T cells without activating regulatory T lymphocytes (Tregs, having immunosuppressive function).

IL-2, a first identified cytokine, was initially found in the supernatant of an activated human T cell culture as a soluble factor that mediated T cell proliferation. IL-2 was also the first FDA-approved cytokine for cancer treatment. Activated dendritic cells (DC), mast cells, and NKT cells also produce a small amount of IL-2, although it is mainly secreted by CD4+ and CD8+ T cells when exposed to antigenic stimulation.

IL-15 and IL-2 have some similar functions, including stimulating proliferation of activated T cells, producing cytotoxic effector T cells, and activating and maintaining NK cells. They also facilitate B cell induced immunoglobulin synthesis and regulated lymphatic homeostasis. Unlike IL-2, however, IL-15 mRNA is expressed in a variety of tissues, including hematopoietic and non-hematopoietic cells, such as keratinocytes, nerve cells, stromal cells and fibroblasts. In contrast to widespread IL-15 mRNA expression, however, mature IL-15 protein production is primarily limited to DC and monocyte/macrophage cells.

IL-15 has a chemotactic effect on T cells: circulating lymphocytes' homing to the peripheral lymph nodes, inhibiting the apoptosis of lymphocytes, promoting the activation and proliferation of T cells, inducing the production of cytotoxic T lymphocytes (CTLs). In the case of CD8+ T cells, apart from promoting the production of memory CD8+ T cells, IL-15 plays a vital role in maintaining the number of in vivo memory CD8+ T cells. In the case of NK cells, IL-15 also plays an important role in its activation and proliferation. In mice overexpressing IL-15, the number of NK cells was significantly increased and the immune response was enhanced. In addition, IL-15 also plays a crucial role in the functional maturation of DC cells and macrophages. In DC cells, IL-15 can facilitate the expression of co-stimulatory factors and IFN-γ by DC cells, and enhance the ability of DC cells to activate CD8+ T cells and NK cells.

IL-2 and IL-15, as type I cytokines with four α-helix bundles, are members of the γ receptor cytokine family. This group of cytokines share the same receptor subunit γc and exhibit pleiotropic effects in regulating both innate and adaptive immune responses. The receptors for both IL-2 and IL-15 are heterotrimers. With exception for the cytokine receptor subunit γc (also known as IL-2Rγ or CD132), they also share a beta subunit, which was referred to herein as IL-2/15Rβ (also referred to as CD122). The third and unique receptor subunits of IL-2 and IL-15 are IL-2Rα and IL-15Rα, respectively.

The α, β, and γ receptors for IL-2 are usually present on the same cell surface. In addition to expression on a cell surface, IL-2Rα is also present in a soluble form (sIL-2Rα) in some diseases, including inflammatory diseases, graft rejection response, and most malignant diseases.

IL-15Rα, as a unique component of an IL-15 receptor complex, is mainly expressed on monocytes and dendritic cells. Unlike other cytokines in the γc family, such as IL-2, cytokine IL-15 first binds to IL-15Rα expressing cells, and then the IL-15/IL-15Rα complex is presented to IL-2/15Rβ and γc on the activated T cells or NK cells to form high-affinity immune synapses. Due to sharing receptor subunits (IL-2/15 Rβγ), IL-2 and IL-15 trigger several similar downstream signalling pathways, including Janus kinase (JAKs) signalling protein and transcriptional activator protein (STATs), in which JAK1 interacts with IL-2/15Rβ, and JAK3 interacts with γc.

Despite these similarities, IL-2 and IL-15 show different functions in vivo, especially in adaptive immune responses. For example, the development and maintenance of regulatory T cells (Tregs) require IL-2, which is closely related to activation-induced cell death (AICD). Treatment of tumors with IL-2 may cause capillary leak syndrome as a side effect. In contrast, IL-15 does not mediate AICD, but inhibits IL-2-induced AICD, nor does it cause capillary leak syndrome as a side effect. IL-15 is the central contributor to the persistence of natural killer (NK) cells and memory CD8+ T cells.

The therapeutic potential of IL-15 has been continually developed in light of its ability to expand and activate effector lymphocytes in vivo. At the same time, the role of IL-15 in the joint strategy for synergizing immune enhancement has attracted increasing attention. N-803 (Anktiva, ALT-803) was formed by the binding of an IL-15 mutant to a sushi region of IL-15/IL-15Rα/IgG1 Fc fusion protein. Compared with a wild-type IL-15, N-803 had good pharmacokinetic properties, longer duration to reside in vivo and stronger anti-tumor activities.

In ASCO 2022, the ImmunityBio released the clinical data to show that in 160 patients with Non-Muscular Invasive Bladder Cancer (NMIBC) without response to BCG, combination of N-803 with Bacillus Calmette Guerin (BCG) resulted in a two-year overall survival of 99%. In a cohort of patients with carcinoma in situ, there was a complete remission of 71%, with a median duration for response of 24.1 months. In a cohort of papillary patient, there was a disease-free survival of 53% at 18 months. Over 90% of patients had avoided cystectomy during a two-year follow-up. The efficacy and safety profile of N-803+BCG in NMIBC patients without response to BCG were superior to other existing therapeutic regimens. Therefore, it will be of positive significance to develop better IL-15 medicaments.

### Summary of the Invention

The technical problem to be resolved by the invention is how to provide a better IL-15 medicament(s).

To solve the technical problem above, the invention provides, in a first aspect, a heterodimer protein comprising protein a and protein b, wherein the protein a comprises an IL-15 mutant and a first Fc mutant; the protein b comprises a sushi domain of an IL-15Rα subunit and a fragment of the other part of the IL-15Rα subunit and a second Fc mutant; the first Fc mutant and the second Fc mutant are selected from a Knob modified Fc or a Hole modified Fc, the Knob modification or the Hole modification in the first Fc mutant and the second Fc mutant is different in type of modification;
the IL-15 mutant is a polypeptide whose amino acid sequence is selected from any one of: positions 1-111 of SEQ ID No.18, positions 1-111 of SEQ ID No.15, positions 1-114 of SEQ ID No.14, positions 1-114 of SEQ ID No.17, positions 1-114 of SEQ ID No.16 and positions 1-114 of SEQ ID No.13; and
the sushi domain of the IL-15Rα subunit and the fragment of the other part of the IL-15Rα subunit is a polypeptide whose amino acid sequence is SEQ ID No.2.

The term "sushi region" in "a sushi domain of an IL-15Rα subunit and a fragment of the other part of the IL-15Rα subunit", as described in the invention, mainly refers to a peptide segment whose amino acid sequence is positions 1-65 of SEQ ID No.2.

The term "fragment of the other part" in "a sushi domain of an IL-15Rα subunit and a fragment of the other part of the IL-15Rα subunit", as described in the invention, mainly refers to a peptide segment whose amino acid sequence is positions 66-78 (DPALVHQRPAPPS) of SEQ ID No.2.

Further, the protein a further comprises a linker which links the IL-15 mutant to the first Fc mutant, wherein the linker can be (GGGGS)n, and n is a natural number from 0 to 4. In one embodiment of the invention, the n is 3.

Still further, the protein a comprises the IL-15 mutant, a linker attached to the C-terminus of the IL-15 mutant, a first Fc mutant attached to the C-terminus of the linker; and the protein b comprises a sushi domain and a fragment of the other part of a IL-15Rα subunit, and a second Fc mutant attached to the C-terminus of the sushi domain and the fragment of the other part of the IL-15Rα subunit.

In the heterodimer protein above, the first Fc mutant or the second Fc mutant may be an Fc of antibody IgG1, IgG2, IgG3, or IgG4, or a mutant thereof.

Further, the first Fc mutant or the second Fc mutant is selected from the Fc of antibody IgG4, or a mutant thereof.

Still further, if a version of IgG4 is used, serine at position 228 in the hinge region thereof may be mutated to proline, to provide a stable IgG4 Fc mutant.

In the heterodimer protein above, the protein a and the protein b are formed by the "Knob into hole" combination in Fc. Among the two peptide chains, the domain of Fc in one chain contains T366W mutation, and that in the other corresponding chain contains T366S, L368A and Y407V mutations; or wherein the domain of Fc in one chain contains T350V, L351Y, F405A and Y407V mutations, and that in the other corresponding chain contains T350V, T360L, K392L and T394V mutations; or a "Knob into hole" combination in otherwise forms.

In one embodiment of the invention, the amino acid sequence of the knob modified Fc (a second Fc mutant) is SEQ ID No.9, and the nucleotide sequence thereof is SEQ ID No.37; and the amino acid sequence of the hole modified Fc (a first Fc mutant) is SEQ ID No.10, and the nucleotide sequence thereof is SEQ ID No.38.

In the heterodimer protein above, the protein a can be any one of A1)-A7):
A1) a protein whose amino acid sequence is SEQ ID No.18;
A2) a protein whose amino acid sequence is SEQ ID No.15;
A3) a protein whose amino acid sequence is SEQ ID No.14;
A4) a protein whose amino acid sequence is SEQ ID No.17;
A5) a protein whose amino acid sequence is SEQ ID No.16;
A6) a protein whose amino acid sequence is SEQ ID No.13;
A7) a fusion protein obtained by attachment of a protein tag to the N-terminus or/and C-terminus of the protein of any one of A1)-A6).

The protein b can be any one of A8)-A10):
A8) a protein whose amino acid sequence is SEQ ID No.19;
A9) a protein whose amino acid sequence is SEQ ID No.20;
A10) a fusion protein obtained by attachment of a protein tag to the N-terminus or/and C-terminus of the protein of A8) or A9).

Preferably, the protein a in the heterodimer protein is a protein whose amino acid sequence is SEQ ID No.18, and the protein b in the heterodimer protein is a protein whose amino acid sequence is SEQ ID No.19.

To solve the technical problem above, the invention provides, in a second aspect, a biological material related to the heterodimer protein above, the biological material is any one of:
B1) a nucleic acid molecule encoding the heterodimer protein above;
B2) an expression cassette comprising the nucleic acid molecule of B1);
B3) a recombinant vector comprising the nucleic acid molecule of B1) or the expression cassette of B2);
B4) a recombinant microorganism comprising the nucleic acid molecule of B1) or the expression cassette of B2) or the recombinant vector of B3);
B5) an animal cell line comprising the nucleic acid molecule of B1) or the expression cassette of B2) or the recombinant vector of B3);
B6) a plant cell line comprising the nucleic acid molecule of B1) or the expression cassette of B2) or the recombinant vector of B3);
B7) a host cell producing the heterodimer protein above.

To solve the technical problem above, the invention provides, in a third aspect, a product having the heterodimer protein above as its active ingredient; wherein use of the product is any of C1)-C6):
C1) prevention and/or treatment of a disease;
C2) activation of the immune system of an organism;
C3) improvement or enhancement of the immune function of an organism;
C4) inhibition of tumor growth;
C5) increase in the proliferation activity of NK cells and/or T cells;
C6) improvement of the ability of NK cells to kill tumor cells, such as K562 cells.

To solve the technical problem above, the invention provides, in a fourth aspect, a pharmaceutical composition consisting of a heterodimer protein as above and additional medicament(s).

Further, the additional medicament(s) can be at least one of: immune checkpoint medicament(s), a bi-specific antibody of a cell adapter type, and a cell therapy product.

To solve the technical problem above, the invention provides, in a fifth aspect, use of the heterodimer protein above or the biological material above or the product above or the pharmaceutical composition above in any one of D1)-D12):
D1) preparing a product for preventing and/or treating a disease;
D2) preparing a product for activating the immune system of an organism;
D3) preparing a product for improving or enhancing the immune function of an organism;
D4) preparing a product for inhibiting tumor growth;
D5) preparing a product for increasing the proliferation activity of NK cells and/or T cells;
D6) preparing a product for improving the ability of NK cells to kill tumor cells, such as K562 cells;
D7) preventing and/or treating a disease;
D8) activating the immune system of an animal;
D9) improving or enhancing the immune function of an organism;
D10) inhibiting tumor growth;
D11) increasing the proliferation activity of NK cells and/or T cells;
D12) improving the ability of NK cells to kill tumor cells, such as K562 cells.

In any of the products or uses above, the T cells comprise CD8+ T cells and/or CD4+ T cells.

In any of the products or uses above, the organism can be a mammal one. The mammal comprises humans and mice.

To solve the technical problem above, the invention provides, in a sixth aspect, a method of treating a disease, comprising: administering to a patient the heterodimer protein above or the biological material above or the product above or the pharmaceutical composition above to treat the patient.

In any of the product or use or method above, the disease comprises infectious disease, tumor, hematological disease, inflammatory disease, and autoimmune disease.

The infectious disease comprises, but is not limited to, viral infection (e.g., smallpox virus infection, HIV infection, HBV infection etc.), bacterial infection, and fungal infection.

The tumor comprises, but is not limited to, melanoma, colorectal cancer, skin cancer, lymphoma, renal cell cancer, liver cancer, lung cancer, gastric cancer, and breast cancer.

The hematological disease comprises, but is not limited to, anemia, acute myeloid leukemia, myelodysplastic syndrome, and T-cell large granular lymphocytic leukemia.

The autoimmune disease comprises, but is not limited to, multiple sclerosis, psoriasis, rheumatic arthritis, gastritis, and mucositis.

In any of the product or use or method above, the product can be medicament(s) or formulation(s). Carrier material(s) can further be added during the preparation of the medicament or formulation in practical application.

The carrier material(s) comprises, but is not limited to, water-soluble carrier materials (e.g., polyethylene glycol, polyvinylpyrrolidone, organic acids etc.), poorly soluble carrier materials (e.g., ethyl cellulose, cholesterol stearate etc.), and enteric carrier materials (e.g., cellulose acetate phthalate, carboxymethyl cellulose etc.).

Various dosage forms can be made with these materials, including, but not limited to tablets, capsules, dripping pills, aerosols, pills, powders, solutions, suspensions, emulsions, granules, liposomes, transdermal agents, buccal tablets, suppositories, lyophilized powders for injection etc. It can be a common formulation, sustained-release formulation, controlled-release formulation and various systems for microparticle administration. Various carriers known in the art can be widely used in preparing the dosage form of unit dosage as tablets . Examples of these carriers are, for example, diluents and absorbents such as starch, dextrin, calcium sulfate, lactose, mannitol, sucrose, sodium chloride, glucose, urea, calcium carbonate, kaolin, microcrystalline cellulose, aluminum silicate, and the like; wetting agents and binding agents such as water, glycerol, polyethylene glycol, ethanol, propanol, starch mucilage, dextrin, syrup, honey, glucose solution, acacia mucilage, gelatin mucilage, sodium carboxymethylcellulose, shellac, methylcellulose, potassium phosphate, polyvinylpyrrolidone, and the like; disintegrating agents such as dried starch, alginate, agar powder, brown algae starch, sodium bicarbonate and citric acid, calcium carbonate, polyoxyethylene, sorbitol fatty acid ester, sodium dodecyl sulfonate, methyl cellulose, ethyl cellulose, and the like; disintegration inhibitors such as sucrose, glycerol tristearate, cocoa butter, hydrogenated oils, and the like; absorption promoters such as quaternary ammonium salts, sodium lauryl sulfate, and the like; lubricants such as powdered talc, silicon dioxide, corn starch, stearate, boric acid, liquid paraffin, polyethylene glycol, and the like. The tablets can be further made into coated tablets such as sugar coated tablets, film coated tablets, enteric coated tablets, or bilayer tablets and multilayer tablets. Various carriers known in the art can be widely used in preparing the dosage form of unit dosage as pills . Examples of these carriers are, for example, diluents and absorbents such as glucose, lactose, starch, cocoa butter, hydrogenated vegetable oils, polyvinylpyrrolidone, kaolin, powdered talc, and the like; binding agents such as acacia gum, tragacanth gum, gelatin, ethanol, honey, liquid sugar, rice paste or batter paste, and the like.; disintegrating agents such as powdered agar, dried starch, alginate, sodium dodecyl sulfonate, methyl cellulose, ethyl cellulose, and the like. Various carriers known in the art can be widely used in preparing the dosage form of unit dosage as suppositories . Examples of these carriers are, for example, polyethylene glycol, lecithin, cocoa butter, higher alcohols, esters of higher alcohols, gelatin, semisynthetic glycerides, and the like. All of the diluents commonly employed in the art, for example, water, ethanol, polyethylene glycol, 1,3-propanediol, ethoxylated isostearyl alcohol, polyoxylated isostearyl alcohol, polyoxyethylene sorbitol fatty acid ester, and the like, can be used in preparing the dosage form of unit dosage for injection , such as solutions, emulsions, lyophilized powders for injection and suspensions. In addition, in order to prepare an isotonic solution for injection, an appropriate amount of sodium chloride, glucose, or glycerol can be added to the formulation for injection, and further, conventional cosolvents, buffers, pH adjusting agents, and the like can be added thereto. Moreover, colorants, preservatives, flavors, flavoring agents, sweeteners, or other materials can be added to the pharmaceutical formulation, if desired.

The dosage forms above can be administrated by injection, including subcutaneous injection, intravenous injection, intramuscular injection, intraluminal injection etc.

The invention provides advantageous technical effects as follows:
1. The instant invention creates, by virtue of computer-assisted design and screening for biological activity, an innovative IL-15 mutant-Fc/IL-15Rα-Fc heterodimer protein, which has a superior biological activity to the current similar products, for example, the dimer protein HL-015-9 as provided by the invention. The dimer protein can stimulate the proliferation of NK cells and T cells well, and can be used alone or in combination with additional medicament(s), such as immune checkpoint medicament(s), a bi-specific antibody of a cell adapter type, or a cell therapy product, to achieve better effects.
2. The heterodimer protein as provided by the invention can increase the proliferative activities of CTLL-2 and Mo7e cells.
3. The heterodimer protein as provided by the invention can increase the proliferative activities of NK cells, CD8+ T cells and CD4+ T cells.
4. The heterodimer protein as provided by the invention can facilitate NK cells to kill K562.
5. The dimer protein, HL-015-9, as provided by the invention, is superior to ALT-803 on the inhibition of growth of tumors at each equivalent injection dose.

### Brief Description of the Drawings

Fig. 1 shows the effects of different molecules from the first round of design on the proliferation of Mo7e cells.
Fig. 2 shows the effects of different molecules from the first round of design on the proliferation of CTLL-2 cells.
Fig. 3 shows the effects of the IL-15 mutant-Fc/IL-15Ralpha fragment-Fc from the second round of design on the proliferation of Mo7e cells.
Fig. 4 shows the effects of the IL-15 mutant-Fc/IL-15Ralpha fragment-Fc from the second round of design on the proliferation of CTLL-2 cells.
Fig. 5 shows the effects of different IL-15 mutant-Fc/IL-15Ralpha fragment-Fc on the proliferation of NK cells.
Fig. 6 shows the effects of different IL-15 mutant-Fc/IL-15Ralpha fragment-Fc on the proliferation of CD4+ T cells.
Fig. 7 shows the effects of different IL-15 mutant-Fc/IL-15Ralpha fragment-Fc on the proliferation of CD8+ T cells.
Fig. 8 shows the stimulation effects of different IL-15 mutant-Fc/IL-15Ralpha fragment-Fc on NK cells' killing effects.
Fig. 9 shows the efficacy of HL-015-9 in a tumor-bearing mouse model.

### Detailed Description of Examples

The invention is described in further detail below in conjunction with particular embodiments, with the examples provided only for illustrating the invention rather than limiting the scope of the invention. The examples provided below can serve as a guide for further modification by one of ordinary skill in the art and are not intended to limit the invention in any way.

The experimental methods in the following examples are conventional ones, unless otherwise specified, and are carried out according to the techniques or conditions described in the literature in the art, or according to the instructions for products. materials, reagents etc. used in the examples described below are commercially available, unless otherwise specified.

### Example 1. Construction of Expression Plasmids of Different Heterodimer Proteins

Different versions of dimer proteins of IL-15/IL-15Rα were designed based on the sequence information of a wild-type IL-15 (SEQ ID No.1) and the sushi domain of the alpha subunit of the IL-15 receptor (positions 1-65 in SEQ ID No.2). According to the information of the amino acid sequences of the designed, different L-15/IL-15Rα dimer proteins, the signal peptide of mouse IL-2 (see SEQ ID No.39 for the amino acid sequence of the signal peptide, and see SEQ ID No.40 for the corresponding nucleotide sequence) and enzymatic cleavage sites were added, and as a result, expression plasmids for different IL-15/IL-15Rα dimer proteins to be secreted by mammalian cells were constructed by GenScript Biotech using conventional gene synthesis and molecular construction techniques.

A heterodimer protein, HL-015-2, was taken as an example to illustrate the process for the construction of a recombinant expression plasmid for eukaryotic cells:
A DNA molecule whose nucleotide sequence was SEQ ID No.28 and a DNA molecule whose nucleotide sequence was SEQ ID No.35 were synthesized by GenScript Biotech. A signal peptide whose nucleotide sequence was SEQ ID No.40 was added upstream of a DNA molecule whose nucleotide sequence was SEQ ID No.28 to provide a new DNA molecule, which was designated as 28'. The fragment between the HindIII and XhoI enzymatic cleavage recognition sites (a small fragment between the HindIII and XhoI enzymatic cleavage recognition sites) in an expression vector pCGS3 (Merck) was replaced with the DNA molecule 28' while remaining the other nucleotide sequences of the expression vector pCGS3 unchanged, resulting in a recombinant expression plasmid, which was designated as pCGS3-015-2a upon confirmation by sequencing. A signal peptide whose nucleotide sequence was SEQ ID No.40 was added upstream of a DNA molecule whose nucleotide sequence was SEQ ID No.35 to provide a new DNA molecule, which was designated as 35'. The fragment between the BstBI and PacI enzymatic cleavage recognition sites (a small fragment between the BstBI and PacI enzymatic cleavage recognition sites) in a recombinant expression plasmid pCGS3-015-2a was replaced with the DNA molecule 35' while remaining the other nucleotide sequences of the recombinant plasmid pCGS3-015-2a unchanged, resulting in a recombinant expression plasmid, which was designated as pCGS3-015-2 upon confirmation by sequencing. The recombinant expression plasmid expressed a protein whose amino acid sequence was SEQ ID No.12 and a protein whose amino acid sequence was SEQ ID No.19, and the two proteins formed a dimer, HL-015-2.

Different heterodimer molecules were constructed from IL-15-Linker Fc fusion and an extracellular region of IL15Rα in different lengths fused with Fc either with or without linker, through "knob into hole"; see SEQ ID No.9 for the information of the amino acid sequence of the knob Fc fragment, whose nucleotide sequence was SEQ ID No.37, and see SEQ ID No.10 for the information of the amino acid sequence of the hole Fc fragment, whose nucleotide sequence was SEQ ID No.38; wherein the amino acid sequences of the heterodimer protein HL-015-1 were SEQ No.11 and SEQ ID No.20, and the nucleotide sequences thereof were SEQ ID No.27 and SEQ ID No.36, respectively; the amino acid sequences of the heterodimer protein HL-015-2 were SEQ No.12 and SEQ ID No.19, and the nucleotide sequences thereof were SEQ ID No.28 and SEQ ID No.35, respectively; and the amino acid sequences of the heterodimer protein HL-015-3 were SEQ No.12 and SEQ ID No.20, and the nucleotide sequences thereof were SEQ ID No.28 and SEQ ID No.36, respectively.

The recombinant expression plasmids HL-015-1 and HL-015-3 were constructed in the same process as described for HL-015-2 except for corresponding replacement with a coding gene of interest.

Positive controls were additionally prepared as follows:
1) Obtaine the coding gene of ALT-803 molecule from the patent CN103370339A (the ALT-803 molecule was designated as ALT-803 in the patent, a dimer consisting of proteins whose amino acid sequences were SEQ ID No.3 and SED ID No.4);
2) With reference to Scientific Reports (2018) 8:7675, a P2239 molecule with IL-15/IL-15Rα Sushi-Fc linked by a disulfide bond was designated as HR-1 (a dimer consisting of a protein whose amino acid sequences were SEQ ID No.5 and SEQ ID No.6);
3) With reference to Scientific Reports (2018) 8:7675, a HRP00018 molecule with IL-15-Fc and IL-15Rα-sushi domain-Fc linked by "Knob into hole" method was designated as HR-2 (a dimer consisting of a protein whose amino acid sequences were SEQ ID No.7 and SEQ ID No.8);

The sequence of the IL-15 in HL-015-1 (positions 1-114 in SEQ ID No.11) was consistent with the sequence of the IL-15 in HR-2 (positions 1-114 in SEQ ID No.7); and there were 4 additional amino acids ( sequence: PAPP) in the sequence of the IL-15Rα subunit in HL-015-1 (positions 1-78 in SEQ ID No.20) than the sequence of the IL-15Rα in HR-2 (positions 1 to 74 in SEQ ID No.8) .

There were 13 more amino acids (the sequence: DPALVHQRPAPPS) in the IL-15Rα in HL-015-2 than the sushi region of the IL-15Rα in ATL-803 (positions 1-65 in SEQ ID No.6).

HL-015-3 differed from HL-015-1 in that the IL-15 in HL-015-3 was introduced a N72D mutation; and HL-015-2 differed from HL-15-03 in that an additional (G4S)3 linker between the IL-15Rα and the Fc was included in HL-015-3.

The designations for the molecules as constructed in the first round according to the invention and amino acid & nucleotide sequences thereof were shown in Table 1.

**Table 1: Designations for the Molecules as Constructed in the First Round and Amino Acid & Nucleotide Sequences thereof**

| Molecule Name | IL-15-Fc | | IL-15Rα-Fc | |
|---|---|---|---|---|
| | Amino Acid Sequence No. | Nucleotide Sequence No. | Amino Acid Sequence No. | Nucleotide Sequence No. |
| HL-015-1 | SEQ ID No.11 | SEQ ID No.27 | SEQ ID No.20 | SEQ ID No.36 |
| HL-015-2 | SEQ ID No.12 | SEQ ID No.28 | SEQ ID No.19 | SEQ ID No.35 |
| HL-015-3 | SEQ ID No.12 | SEQ ID No.28 | SEQ ID No.20 | SEQ ID No.36 |
| ALT-803 | SEQ ID No.3 | SEQ ID No.21 | SEQ ID No.4 | SEQ ID No.22 |
| HR-1 | SEQ ID No.5 | SEQ ID No.23 | SEQ ID No.6 | SEQ ID No.24 |
| HR-2 | SEQ ID No.7 | SEQ ID No.25 | SEQ ID No.8 | SEQ ID No.26 |

### Example 2. Expression & Purification of Proteins

The recombinant expression plasmids pCGS3-015-1, pCGS3-015-2, pCGS3-015-3, pCGS3-ALT-803, pCGS3-HR-1 and pCGS3-HR-2 in Example 1 were respectively transfected, with each plasmid transfected into an amount of 200 mL of culture at a plasmid level of 0.7 µg/mL and incubated for 10 days, according to the instructions for use of ExpiCHO^{™} Expression System Kit available from Thermofisher. The cell density increased significantly at the initial stage during the incubation, and then decreased slowly, with high cell viability maintained. Cell viability of 80% was kept when samples were collected on Day 10.

The supernatant was collected by centrifugation, suction filtered and then purified by MabSelect SuRe LX (available from Cytiva, catalog number 17-5474-02) (CIP buffer: 0.1M NaOH, equilibration buffer: 20 mM PB, 0.15 M NaCl, pH 7.0, washing buffer 1: 20 mM PB, 1 M NaCl, pH 7.0, washing buffer 2: 50 mM citric acid-sodium citrate, pH 5.5, elution buffer: 50 mM citric acid-sodium citrate, pH 3.5); after the eluents were collected and concentrated, they were subjected to further purification at a loading volume of 3%, onto Superdex 200 (available from Cytiva, catalog number V521276) (equilibration & elution solution: 20 mmol/L PB, 150 mmol/L NaCl, pH7.0); desired peaks were collected and purity was detected by SEC-HPLC, and concentrations of the proteins were determined using a UV spectrophotometer and according to the theoretical extinction coefficient of each protein. HL-015-1 (expressed by the recombinant expression plasmid pCGS3-015-1), HL-015-2 (expressed by the recombinant expression plasmid pCGS3-015-2), HL-015-3 (expressed by the recombinant expression plasmid pCGS3-015-3), ALT-803 (expressed by the recombinant expression plasmid pCGS3-ALT-803), HR-1 (expressed by the recombinant expression plasmid pCGS3-HR-1), and HR-2 (expressed by the recombinant expression plasmid pCGS3-HR-2) were obtained as the heterodimer proteins.

### Example 3. Assay for Effects of Heterodimer Proteins on Proliferation Activities of CTLL-2 and Mo7e Cells

Cytokine dependent (for growth) CTLL2 cells (Suzhou Vertex Biotech Co., Ltd., catalog number TCM-C724) were cultured in a RPMI 1640 medium supplemented with 200 U/mL IL-2 and 10% fetal bovine serum at 37°C, 5.0% CO₂ to a logarithmic phase, then centrifuged at 1000 rpm for 5 minutes to harvest cells, and washed with phosphate buffered saline for three times. The cells were then re-suspended in a RPMI 1640 medium containing 10% fetal bovine serum (the assay medium), added to a 96-well cell culture plate at a density of 5,000 cells per well and cultured for 4 hours (cytokine starvation), followed by gradient dilution of the heterodimer proteins HL-015-1, HL-015-2, HL-015-3, ALT-803, HR-1 and HR-2 in Example 2 with PBS, respectively, to provide solutions of heterodimer proteins, 10 µl of which was added to each well. Each heterodimer protein was set to treatments of 11 concentrations, the heterodimer proteins in 11 wells had a concentration of 500 ng/ml, 166.67 ng/ml, 55.56 ng/ml, 18.52 ng/ml, 6.17 ng/ml, 2.06 ng/ml, 0.69 ng/ml, 0.23 ng/ml, 0.076 ng/ml, 0.025 ng/ml and 0.0085 ng/ml, respectively, and a negative control was set with 10 µl of PBS added for each well, (i.e., 0 ng/ml). Duplicate wells were configured for each concentration. Following an additional culture for 2 days, CCK8 was added and the cells were cultured at 37°C, 5.0% CO₂ for 2 hours. The plates were then read at 450 nm and 630 nm for cell growth. Triplicates were set for each experiment.

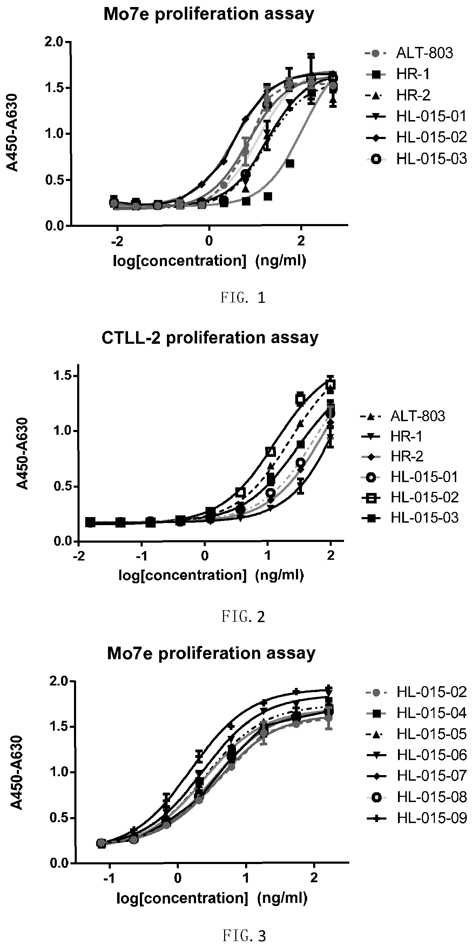

A similar method to CTLL-2 was carried out for the test of the stimulation of IL-15/IL-15Rα dimer proteins on the proliferation of Mo7e cells (Zhejiang Meisen Cell Technology Co., Ltd., catalog number CTCC-001-0368). Mo7e cells were cultured to a logarithmic phase in a RPMI 1640 medium supplemented with 8 ng/ml GM-CSF and 10% fetal bovine serum. Cells were harvested and washed, then re-suspended in a RPMI 1640 medium free of GM-CSF, and added to a 96-well plate at a density of 20,000 cells per well. After 4 hours of culture, the heterodimer proteins HL-015-1, HL-015-2, HL-015-3, ALT-803, HR-1, and HR-2 in Example 2 were subjected to gradient dilution with PBS, respectively, to provide solutions of heterodimer proteins, 10 µl of which was added to each well. Each heterodimer protein was set to treatments of 8 concentrations, the heterodimer proteins in these 8 wells had a concentration of 100 ng/ml, 33.33 ng/ml, 11.11 ng/ml, 3.70 ng/ml, 1.23 ng/ml, 0.41 ng/ml, 0.046 ng/ml, and 0.015 ng/ml, respectively, and a negative control was set with 10 µl of PBS added for each well (i.e., 0 ng/ml). Duplicate wells were configured for each concentration, and the cells were cultured at 37°C, 5.0% CO₂ for 4 days. 4 days later, CCK8 was added (20 µl/well) and the cells were cultured at 37°C, 5.0% CO₂ for 2 hours. The plates were then read at 450 nm and 630 nm for cell growth. Triplicates were set for each experiment. The results showed that HL-015-2 had optimal ability to stimulate the proliferation of CTLL-2 or Mo7e cells (the results were shown in Figs. 1 and 2, with logarithmic values base 10 in Figs. 1 and 2). HL-015-1 was superior to HR-2 with the main difference being that there were 4 more amino acids in the IL-15Rα subunit in HL-015-1 than in the IL-15Rα in HR-2. Also, there were 13 more amino acids (DPALVHQRPAPPS) in the IL-15Rα in HL-015-02 than that of the sushi region (positions 1-65 in SEQ ID No.6) of the IL-15Rα in ATL-803.

### Example 4. Designs for IL-15 Mutants and Acquisition of Heterodimer Proteins and Activity Comparison

In view of the experimental results from Examples 2 and 3, the inventors decided to choose the IL-15 N72D mutation as set forth in SEQ ID No.3 and the IL-15Rα-Fc as set forth in SEQ ID No.19 for use in further experiments as described below.

IL-15, IL-15Rα and IL-15Rβ/γ were subjected to analysis for their structures using relevant software for structural analysis to identify amino acid sites that might affect IL-15, and computer modelling and molecular docking were performed to select amino acid mutations that might enhance the activity of IL-15, and corresponding genes were synthesized. The amino acid mutation sites obtained by screening were shown in Table 2.

**Table 2: IL15 Mutants and Amino Acid Sequences Thereof**

| Type of Mutation | Amino Acid Sequence No. | Amino Acid Sequence |
|---|---|---|
| IL15 N72D/L45E/L52E | Sequence 13 Positions 1-114 | NWVNVISDLKKIEDLIQSMH IDATLYTESDVHPSCKVTAM KCFLEELQVISEESGDASIHD TVENLIILANDSLSSNGNVTE SGCKECEELEEKNIKEFLQSF VHIVQMFINTS |
| IL15 N72D/L45D/L52D | Sequence 14 Positions 1-114 | NWVNVISDLKKIEDLIQSMH IDATLYTESDVHPSCKVTAM KCFLDELQVISDESGDASIHD TVENLIILANDSLSSNGNVTE SGCKECEELEEKNIKEFLQSF VHIVQMFINTS |
| IL15 N72D/L45E/L52D | Sequence 17 Positions 1-114 | NWVNVISDLKKIEDLIQSMH IDATLYTESDVHPSCKVTAM KCFLEELQVISDESGDASIHD TVENLIILANDSLSSNGNVTE SGCKECEELEEKNIKEFLQSF VHIVQMFINTS |
| IL15 N72D/L45D/L52E Mutant | Sequence 16 Positions 1-114 | NWVNVISDLKKIEDLIQSMH IDATLYTESDVHPSCKVTAM KCFLDELQVISEESGDASIHD TVENLIILANDSLSSNGNVTE SGCKECEELEEKNIKEFLQSF VHIVQMFINTS |
| IL15 Mutant Containing N72D, with 3 Amino Acids (NTS) Deleted from C-Terminus | Sequence 15 Positions 1-111 | NWVNVISDLKKIEDLIQSMH IDATLYTESDVHPSCKVTAM KCFLLELQVISLESGDASIHD TVENLIILANDSLSSNGNVTE SGCKECEELEEKNIKEFLQSF VHIVQMFI |
| IL15 Mutant Containing N72D/L45D/L52D , with 3 Amino Acids (NTS) Deleted from C-Terminus | Sequence 18 Positions 1-111 | NWVNVISDLKKIEDLIQSMH IDATLYTESDVHPSCKVTAM KCFLDELQVISDESGDASIHD TVENLIILANDSLSSNGNVTE SGCKECEELEEKNIKEFLQSF VHIVQMFI |

A DNA molecule whose nucleotide sequence was SEQ ID No.34 and a DNA molecule whose nucleotide sequence was SEQ ID No.35 were synthesized. A signal peptide whose nucleotide sequence was SEQ ID No.40 was added upstream of the DNA molecule whose nucleotide sequence was SEQ ID No.34 to provide a new DNA molecule, which was designated as 34'. The fragment between the HindIII and XhoI enzymatic cleavage recognition sites (a small fragment between the HindIII and XhoI enzymatic cleavage recognition sites) in an expression vector pCGS3 (Merck) was replaced with the DNA molecule 34' while remaining the other nucleotide sequences of the expression vector pCGS3 unchanged, resulting in a recombinant expression plasmid, which was designated as pCGS3-015-9a upon confirmation by sequencing. A signal peptide whose nucleotide sequence was SEQ ID No.40 was added upstream of the DNA molecule whose nucleotide sequence was SEQ ID No.35 to provide a new DNA molecule, which was designated as 35'. The fragment between the BstBI and PacI enzymatic cleavage recognition sites (the small fragment between the BstBI and PacI enzymatic cleavage recognition sites) in the recombinant expression plasmid pCGS3-015-9a was replaced with the DNA molecule 35' while remaining the other nucleotide sequences of the recombinant plasmid pCGS3-015-9a unchanged, resulting in a recombinant expression plasmid, which was designated as pCGS3-015-9 upon confirmation by sequencing. The recombinant expression plasmid pCGS3-015-9 contained a HL-015-9 gene (a DNA molecule whose nucleotide sequence was SEQ ID No.34 and a DNA molecule whose nucleotide sequence was SEQ ID No.35) and expressed a protein whose amino acid sequence was SEQ ID No.18 and a protein whose amino acid sequence was SEQ ID No.19, and the two proteins formed a heterodimer protein, HL-015-9.

Recombinant expression plasmids of HL-015-4 to HL-015-8 were constructed in the same method as described for HL-015-9 except for a corresponding substitution for the HL-015-9 gene, resulting in a recombinant expression plasmid pCGS3-HL-015-4 for the HL-015-4 gene (whose nucleotide sequences were SEQ ID No.29 and SEQ ID No.35, respectively), a recombinant expression plasmid pCGS3-HL-015-5 for the HL-015-5 gene (whose nucleotide sequences were SEQ ID No.30 and SEQ ID No.35, respectively), a recombinant expression plasmid pCGS3-HL-015-6 for the HL-015-6 gene (whose nucleotide sequences were SEQ ID No.31 and SEQ ID No.35, respectively), a recombinant expression plasmid pCGS3-HL-015-7 for the HL-015-7 gene (whose nucleotide sequence were SEQ ID No.32 and SEQ ID No.35, respectively), and a recombinant expression plasmid PCGS3-HL-015-8 for the HL-015-8 gene (whose nucleotide sequences were SEQ ID No.33 and SEQ ID No.35, respectively). pCGS3-HL-015-4 expressed a protein whose amino acid sequence was SEQ ID No.13 and a protein whose amino acid sequence was SEQ ID No.19, and the two proteins formed a heterodimer protein HL-015-4. pCGS3-HL-015-5 expressed a protein whose amino acid sequence was SEQ ID No.14 and a protein whose amino acid sequence was SEQ ID No.19, and the two proteins formed a heterodimer protein HL-015-5. pCGS3-HL-015-6 expressed a protein whose amino acid sequence was SEQ ID No.15 and a protein whose amino acid sequence was SEQ ID No.19, and the two proteins formed a heterodimer protein HL-015-6. pCGS3-HL-015-7 expressed a protein whose amino acid sequence was SEQ ID No.16 and a protein whose amino acid sequence was SEQ ID No.19, and the two proteins formed a heterodimer protein HL-015-7. pCGS3-HL-015-8 expressed a protein whose amino acid sequence was SEQ ID No.17 and a protein whose amino acid sequence was SEQ ID No.19, and the two proteins formed a heterodimer protein HL-015-8.

The designations for and amino acid & nucleotide sequences of the molecules as constructed in the second round according to the invention were shown in Table 3.

**Table 3: Designations for and Amino Acid & Nucleotide Sequences of the Molecules as Constructed in the Second Round**

| Designation for Molecule | IL-15 Mutant-Fc | | IL-15Rα-Fc | |
|---|---|---|---|---|
| | Amino Acid Sequence No. | Nucleotide Sequence No. | Amino Acid Sequence No. | Nucleotide Sequence No. |
| HL-015-4 | SEQ ID No.13 | SEQ ID No.29 | SEQ ID No.19 | SEQ ID No.35 |
| HL-015-5 | SEQ ID No.14 | SEQ ID No.30 | SEQ ID No.19 | SEQ ID No.35 |
| HL-015-6 | SEQ ID No.15 | SEQ ID No.31 | SEQ ID No.19 | SEQ ID No.35 |
| HL-015-7 | SEQ ID No.16 | SEQ ID No.32 | SEQ ID No.19 | SEQ ID No.35 |
| HL-015-8 | SEQ ID No.17 | SEQ ID No.33 | SEQ ID No.19 | SEQ ID No.35 |
| HL-015-9 | SEQ ID No.18 | SEQ ID No.34 | SEQ ID No.19 | SEQ ID No.35 |

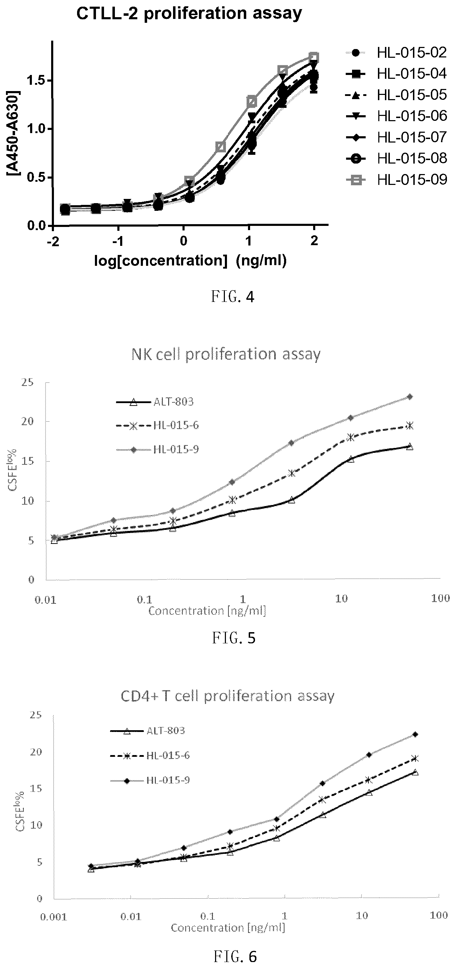

The recombinant expression plasmids pCGS3-HL-015-4 to pCGS3-HL-015-9 were expressed and proteins purified according to the method described for Example 2. Assay for activity was then performed according to the method described for Example 3, and the results were shown in Figs. 3 and 4 (with logarithmic values base 10 in Figs. 3 and 4). Among all the mutants, HL-015-9 was the most active one in stimulating CTLL-2 proliferation or Mo7e proliferation.

In the experiment of the proliferation of CTLL-2 cells, according to the different types of heterodimer proteins, the heterodimer proteins HL-015-4, HL-015-5, HL-015-6, HL-015-7, HL-015-8 and HL-015-9 were subjected to gradient dilution with PBS, respectively, to get solutions of heterodimer proteins, 10 µl of which was added to each well. Each heterodimer protein was set to treatments of 8 concentrations, the heterodimer proteins in these 8 wells had the concentrations of 166.67 ng/ml, 55.56 ng/ml, 18.52 ng/ml, 6.17 ng/ml, 2.06 ng/ml, 0.69 ng/ml, 0.23 ng/ml, and 0.076 ng/ml respectively. Triplicates were set for each experiment, and a negative control was set with 10 µl of PBS added for each well (i.e., 0 ng/ml), and duplicate wells were configured for each concentration. The remaining operations were the same as that in Example 3.

In the experiment of the proliferation of the Mo7e cells, according to the different types of heterodimer proteins, the heterodimer proteins HL-015-4, HL-015-5, HL-015-6, HL-015-7, HL-015-8 and HL-015-9 were subjected to gradient dilution with PBS, to get solutions of heterodimer proteins, 10 µl of which was added to each well. Each heterodimer protein was set to treatments of 8 concentrations, the heterodimer proteins in these 8 wells had the concentrations of 100 ng/ml, 33.33 ng/ml, 11.11 ng/ml, 3.70 ng/ml, 1.23 ng/ml, 0.41 ng/ml, 0.046 ng/ml and 0.015ng/ml , respectively. Triplicates were set for each experiment, and a negative control was set with 10 µl of PBS added for each well (i.e., 0 ng/ml), and duplicate wells were configured for each concentration. The remaining operations were the same as that in Example 3.

### Example 5. Stimulation Effects of Heterodimer Proteins on Proliferation of NK Cells, CD8+ T Cells and CD4+ T Cells

NK cells (CD56+, CD3-), CD8+ T cells (CD3+, CD8+) and CD4+ T cells (CD3+, CD8+) were isolated from human peripheral blood cells using kits for isolation of NK cells, CD8+ T cells and CD4+ T cells, respectively. The isolated cells were cultured overnight at 37°C, 5.0% CO₂ in a RPMI 1640 medium containing 10% fetal bovine serum, and the cells were harvested for staining with Carboxyfluorescein Diacetate Succinimidyl Ester (CFSE) and incubated for 15 minutes at 37°C. Then they were centrifuged, washed, and re-suspended in a RPMI 1640 medium containing 10% fetal bovine serum, followed by addition, at a density of 10,000 cells per well, to a 96-well cell culture plate pre-filled with heterodimer protein solutions of ALT-803, HL-015-6 and HL-015-9, respectively, at different concentrations obtained by dilution with PBS. After 3 days of culture, the number of CFSC-positive cells was assayed on a flow cytometer to determine the effects of different IL-15/IL-15Rα heterodimers on the growth of different cells. As shown in Figs. 5, 6 and 7, HL-015-9 had the strongest activity to stimulate the proliferation of NK cells, CD4+ T cells and CD8+ T cells.

In the experiment of the proliferation of NK cells, according to the different types of dimer proteins, the experiments were divided into three groups. Upon gradient dilution with PBS, the concentrations of heterodimer proteins ALT-803, HL-015-6 and HL-015-9 were at 50 ng/ml, 12.5 ng/ml, 3.13 ng/ml, 0.78 ng/ml, 0.20 ng/ml, 0.049 ng/ml, 0.012 ng/ml and 0.031 ng/ml, respectively. Triplicates were set for each experiment, and a negative control was set with 10 µl of PBS added for each well (i.e., 0 ng/ml), and duplicate wells were set for each concentration.

In the experiment of the proliferation of CD4+ cells, according to the different types of dimer proteins, the experiments were divided into three groups. Upon gradient dilution with PBS, the concentrations of heterodimer proteins ALT-803, HL-015-6 and HL-015-9 were at 50 ng/ml, 12.5 ng/ml, 3.13 ng/ml, 0.78 ng/ml, 0.20 ng/ml, 0.049 ng/ml, 0.012 ng/ml, 0.031 ng/ml and 0 ng/ml, respectively. Triplicates were set for each experiment, and a negative control was set with 10 µl of PBS added for each well (i.e., 0 ng/ml), and duplicate wells were set for each concentration.

In the experiment of the proliferation of CD8+ cells, according to the different types of dimer proteins, the experiments were divided into three groups. Upon gradient dilution with PBS, the concentrations of heterodimer proteins ALT-803, HL-015-6 and HL-015-9 were at 50 ng/ml, 12.5 ng/ml, 3.13 ng/ml, 0.78 ng/ml, 0.20 ng/ml, 0.049 ng/ml, 0.012 ng/ml, 0.031 ng/ml and 0 ng/ml, respectively. Triplicates were set for each experiment, and a negative control was set with 10 µl of PBS added for each well (i.e., 0 ng/ml), and duplicate wells were set for each concentration.

### Example 6. Effects of Heterodimer Proteins on Killing of K562 by NK Cells

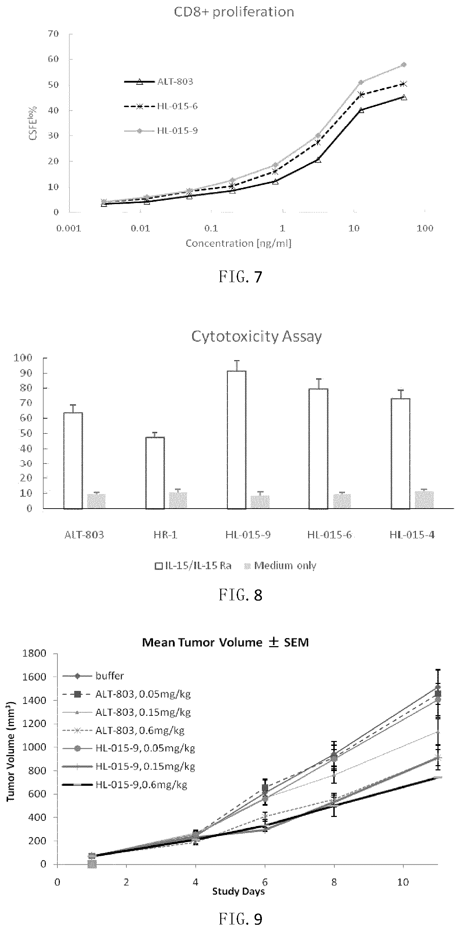

K562 cells (Wuhan Procell, catalog number CL-0130) were cultured in a RPMI 1640 medium containing 5% fetal bovine serum at 37°C, 5.0% CO₂ for 48 hours, to which a solution of BATDA dye was then added for incubation at 37°C for 25 minutes, followed by centrifugation and washing with the medium for three times. The labelled K562 cells were added to a 96-well culture plate at a density of 5,000 cells per well. The NK cells, which were co-cultured in advance for 72 hours in a medium (a RPMI 1640 medium containing 5% fetal bovine serum) with the heterodimer proteins ALT-803, HR-1, HL-015-9, HL-015-6, and HL-015-4 at a final concentration of 100 ng/ml, were added, respectively, at a target cell/effector cell ratio of 1:10. The target cells and effector cells were incubated at 37°C, 5.0% CO₂ for 4 hours, followed by centrifugation of the supernatant and addition of a solution of europium to detect the fluorescence value of the solution, with NK cells cultured with the medium alone as a control. At the same time, a blank control group and a maximum release group were established (the maximum release group was formed by addition of Triton X at a final concentration of 0.05% to the blank control group, detected by added europium following incubation). The killing rate was calculated according to the formula as follows: killing rate (%) = (average of experimental group - average of blank control group) / (average of maximum release group - average of blank control group). The experimental results were shown in Fig. 8. Each of the IL-15/IL-15Rα complexes was able to substantially increase the killing of K562 by NK cells, among which HL-015-9 was the most active one.

### Example 7. In vivo Pharmacodynamic Evaluation of Heterodimer Proteins in Animals

BALB/c mice (7-9-week-old females, weighing from 17 to 23 g, purchased from Beijing Vital River Laboratory Animal Technology Co., Ltd., Animal Batch No. 110011211108488925) were subcutaneously inoculated with CT26 cells (a cell line of mouse colon cancer cultured in a RPMI1640 culture solution containing 10% fetal bovine serum and preserved at Crown Biocience) to establish a mouse tumor model with colon cancer subcutaneously grafted. By the time the tumor volume reached 60-100 cubic millimeters, the mice were randomly grouped and administered (the experiment was divided into seven groups, with seven tumor-bearing mice in each group). The first day of administration was Day 1, and the administration cycles covered Day 1, Day 4, Day 8 and Day 11. The mice were administered with a volume of 10 µl/g body weight and measured for tumor volume twice a week, and the tumor volume was calculated according to the formula as follows: tumor volume (mm³) = 1/2 × (a × b²) (wherein a represents the major diameter and b represents the minor diameter).

The heterodimer protein ALT-803 was dissolved in a buffer to provide a solution of heterodimer protein ALT-803; and heterodimer protein HL-015-9 was dissolved in a buffer to provide a solution of heterodimer protein HL-015-9; wherein the buffer was 0.9% normal saline for injection.

In the experiment, a control group was a buffer group, and each mouse was injected intraperitoneally with the buffer at a volume of 10 µl/g body weight.

Group 1: ALT-803, 0.05 mg/kg. Each mouse was intraperitoneally injected with a solution of heterodimer protein ALT-803 at a volume of 10 µl/g body weight such that the heterodimer protein ALT-803 was administered in a dose of 0.05 mg/kg body weight.

Group 2: ALT-803, 0.15 mg/kg. Each mouse was intraperitoneally injected with a solution of heterodimer protein ALT- 803 at a volume 10 µl/g body weight such that the heterodimer protein ALT-803 was administered in a dose of *0.15* mg/kg body weight.

Group 3: ALT-803, 0.6 mg/kg. Each mouse was intraperitoneally injected with a solution of heterodimer protein ALT-803 at a volume of 10 µl/g body weight such that the heterodimer protein ALT-803 was administered in a dose of 0.6 mg/kg body weight.

Group 4: HL-015-9, 0.05 mg/kg. Each mouse was intraperitoneally injected with a solution of heterodimer protein HL-015-9 at a volume of 10 µl/g body weight such that the heterodimer protein HL-015-9 was administered in a dose of 0.05 mg/kg body weight.

Group 5: HL-015-9, 0.15 mg/kg. Each mouse was intraperitoneally injected with a solution of heterodimer protein HL-015-9 at a volume of 10 µl/g body weight such that the heterodimer protein HL-015-9 was administered in a dose of 0.15 mg/kg body weight.

Group 6: HL-015-9, 0.6 mg/kg. Each mouse was intraperitoneally injected with a solution of heterodimer protein HL-015-9 at a volume of 10 µl/g body weight such that the heterodimer protein HL-015-9 was administered in a dose of 0.6 mg/kg body weight.

The results were shown in Fig. 9. The results showed that at the same concentration, HL-015-9 was superior to ALT- 803 at each corresponding concentration in terms of inhibiting tumor growth in the model mice.

The invention has been described in detail above. It will be apparent to those skilled in the art that the invention can be practiced within a wider range using equivalent parameters, concentrations and conditions without departing from the spirit and scope of the invention and without unnecessary experimentation. While the invention has been described with respect to specific embodiments, it should be understood that the invention can be further modified. In summary, according to the principles of the invention, this application is intended to encompass any alterations, uses, or modifications to the invention, including those departing from the scope disclosed in this application but resulting from conventional techniques known in the art. Some basic features may be applied within the scope contained in the following appended claims.

### Industrial Application

The invention provides an IL-15 mutant-Fc/IL-15Rα subunit-Fc heterodimer protein comprising protein a and protein b, wherein the protein a can be a protein whose amino acid sequence is SEQ ID No.18 and the protein b can be a protein whose amino acid sequence is SEQ ID No.19. It had been shown in experiments that the IL-15 mutant-Fc/IL-15Rα subunit-Fc heterodimer protein and the relevant biological material as provided by the invention can effectively stimulate the proliferation of NK cells and T cells, and in vivo experiments showed that the heterodimer protein HL-015-9 was superior to ALT-803 in terms of inhibiting growth of tumors at each equivalent injected dose. Thus, the IL-15 mutant-Fc/IL-15Rα subunit-Fc heterodimer protein as provided by the invention can be used for preparing medicaments or formulations for treating tumors and/or viral infections.

## Claims

1. A heterodimer protein comprising protein a and protein b, wherein the protein a comprises an IL-15 mutant and a first Fc mutant; the protein b comprises a sushi domain of an IL-15Rα subunit and a fragment of the other part of the IL-15Rα subunit and a second Fc mutant; the first Fc mutant and the second Fc mutant are selected from a Knob modified Fc or a Hole modified Fc, and the Knob modification or the Hole modification in the first Fc mutant and the second Fc mutant is different in type of modification;
wherein the IL-15 mutant is a polypeptide whose amino acid sequence selected from any of : positions 1-111 of SEQ ID No.18, positions 1-111 of SEQ ID No.15, positions 1-114 of SEQ ID No.14, positions 1-114 of SEQ ID No.17, positions 1-114 of SEQ ID No.16 and positions 1-114 of SEQ ID No.13; and
wherein the sushi domain of the IL-15Rα subunit and the fragment of the other part of the IL-15Rα subunit is a polypeptide whose amino acid sequence is SEQ ID No.2.

2. The heterodimer protein according to claim 1, **characterized in that** the protein a further comprises a linker which links the IL-15 mutant to the first Fc mutant, wherein the linker is (GGGGS)n, and n is a natural number from 0 to 4.

3. The heterodimer protein according to claim 2, **characterized in that** the n is 3.

4. The heterodimer protein according to any of claims 1-3, wherein the protein a comprises: the IL-15 mutant, a linker attached to the C-terminus of the IL-15 mutant, a first Fc mutant attached to the C-terminus of the linker; and the protein b comprises: a sushi domain and a fragment of the other part of the IL-15Rα subunit, and a second Fc mutant attached to the C-terminus of the sushi domain of the IL-15Rα subunit and the fragment of the other part of the IL-15Rα subunit.

5. The heterodimer protein according to any of claims 1-4, **characterized in that** the first Fc mutant or the second Fc mutant is an Fc of antibody IgG1, IgG2, IgG3, or IgG4, or a mutant thereof.

6. The heterodimer protein according to any of claims 1-5, **characterized in that** the first Fc mutant or the second Fc mutant is the Fc of antibody IgG4, or a mutant thereof.

7. The heterodimer protein according to any of claims 1-6, **characterized in that** the amino acid sequence of the Knob modified Fc is SEQ ID No.9.

8. The heterodimer protein according to any of claims 1-7, **characterized in that** the amino acid sequence of the Hole modified Fc is SEQ ID No.10.

9. The heterodimer protein according to any of claims 1-8, **characterized in that** the protein a is any one of A1) to A7):
A1) a protein whose amino acid sequence is SEQ ID No.18;
A2) a protein whose amino acid sequence is SEQ ID No.15;
A3) a protein whose amino acid sequence is SEQ ID No.14;
A4) a protein whose amino acid sequence is SEQ ID No.17;
A5) a protein whose amino acid sequence is SEQ ID No.16;
A6) a protein whose amino acid sequence is SEQ ID No.13;
A7) a fusion protein resulting from attachment of a protein tag to the N-terminus or/and C-terminus of the protein of any of A1)-A6).

10. The heterodimer protein according to any of claims 1-9, **characterized in that** the protein b is any one of A8)-A10):
A8) a protein whose amino acid sequence is SEQ ID No.19;
A9) a protein whose amino acid sequence is SEQ ID No.20;
A10) a fusion protein resulting from attachment of a protein tag to the N-terminus or/and C-terminus of the protein of A8) or A9).

11. A biological material related to the heterodimer protein according to any of claims 1-10, **characterized in that** the biological material is any one of:
B1) a nucleic acid molecule encoding the heterodimer protein of any of claims 1-10;
B2) an expression cassette comprising the nucleic acid molecule of B1);
B3) a recombinant vector comprising the nucleic acid molecule of B1) or the expression cassette of B2);
B4) a recombinant microorganism comprising the nucleic acid molecule of B1) or the expression cassette of B2) or the recombinant vector of B3);
B5) an animal cell line comprising the nucleic acid molecule of B1) or the expression cassette of B2) or the recombinant vector of B3);
B6) a plant cell line comprising the nucleic acid molecule of B1) or the expression cassette of B2) or the recombinant vector of B3);
B7) a host cell producing the heterodimer protein of any of claims 1-10.

12. A product having the heterodimer protein according to any of claims 1-10 as its active ingredient;
wherein the product has a use of any of C1)-C6):
C1) prevention and/or treatment of a disease;
C2) activation of the immune system of an organism;
C3) improvement or enhancement of the immune function of an organism;
C4) inhibition of tumor growth;
C5) increase in the proliferative activity of NK cells and/or CD8+T cell and/or CD4+ T cells;
C6) improvement of the ability of NK cells to kill tumor cells.

13. A pharmaceutical composition consisting of the heterodimer protein according to any of claims 1-10 and additional medicament(s).

14. The pharmaceutical composition of claim 13, **characterized in that** the additional medicament(s) can be at least one of an immune checkpoint medicament, a bi-specific antibody of a cell adapter type, and a cell therapy product.

15. Use of the heterodimer protein of any of claims 1-10, the biological material of claim 11, the product of claim 12, or the pharmaceutical composition of claim 13 or 14 in any one of D1)-D12):
D1) preparing a product for preventing and/or treating a disease;
D2) preparing a product for activating the immune system of an organism;
D3) preparing a product for improving or enhancing the immune function of an organism;
D4) preparing a product for inhibiting tumor growth;
D5) preparing a product for increasing the proliferative activity of NK cells and/or T cells;
D6) preparing a product for improving the ability of NK cells to kill tumor cells;
D7) preventing and/or treating a disease;
D8) activating the immune system of an animal;
D9) improving or enhancing the immune function of an organism;
D10) inhibiting tumor growth;
D11) increasing the proliferative activity of NK cells and/or T cells;
D12) improving the ability of NK cells to kill tumor cells.

16. The method according to claim 15, **characterized in that** the disease comprises infectious disease, tumor, hematological disease, inflammatory disease, and autoimmune disease.

17. A method of treating a disease comprising a step of administering to a patient the heterodimer protein of any of claims 1-10, the biological material of claim 11 ,the product of claim 12, or the pharmaceutical composition of claim 13 or 14 to treat the patient.

18. The method of claim 17, **characterized in that** the disease comprises infectious disease, tumor, hematological disease, inflammatory disease, and autoimmune disease.
